# EUROPÄISCHE PATENTSCHRIFT

(11) **EP 0 980 274 B1**
(45) Veröffentlichungstag und Bekanntmachung des Hinweises auf die Patenterteilung: **14.05.2003**
(21) Anmeldenummer: 98928220.7
(22) Anmeldetag: 29.04.1998
(51) Int. Cl.: A61L 31/00, A61K 51/12

(54) **POLYMERBESCHICHTETE STENTS, VERFAHREN ZU IHRER HERSTELLUNG UND IHRE VERWENDUNG ZUR RESTENOSEPROPHYLAXE**
POLYMER-COATED STENTS, PROCESSES FOR PRODUCING THE SAME AND THEIR USE FOR RESTENOSIS PREVENTION
TUTEURS A REVETEMENT POLYMERE, LEURS PROCEDES DE FABRICATION ET LEUR UTILISATION EN PROPHYLAXIE DE LA RESTENOSE

(30) Priorität: 30.04.1997 DE 19718339
(43) Veröffentlichungstag der Anmeldung: 23.02.2000
(60) Teilanmeldung: 03090026.0
(73) Patentinhaber: SCHERING AKTIENGESELLSCHAFT, 13353 Berlin (DE)
(72) Erfinder: KRAUSE, Werner, D-13505 Berlin (DE); HÖCKER, Hartwig, D-52076 Aachen (DE); LAHANN, Jörg, D-52064 Aachen (DE); KLEE, Doris, D-52074 Aachen (DE)
(86) Internationale Anmeldenummer: EP9802528
(87) Internationale Veröffentlichungsnummer: WO98048852

(56) Entgegenhaltungen:
- EP-A- 0 433 011
- EP-A- 0 819 446
- WO-A-97/38730
- DE-C- 4 315 002

## Beschreibung

Die Erfindung betrifft polymerbeschichtete Stents, Verfahren zu ihrer Herstellung und ihre Verwendung zur Restenoseprophylaxe.

### Stand der Technik

Stents sind Stand der Technik (Pschyrembel, Klinisches Wörterbuch, 257. Auflage, Verlag W. de Gruyter). Stents sind selbstexpandierende Endoprothesen, die die Offenhaltung gangartiger Strukturen in Körpern von Menschen oder Tieren ermöglichen (z.B. Gefäß-, Ösophagus-, Trachea-, Gallengangstent). Sie werden als palliative Maßnahme bei Verengungen durch Verschluß (z.B. Atherosklerose) oder Druck von außen (z.B. bei Tumoren) verwendet. Radioaktive Stents werden beispielsweise nach gefäßchirurgischen oder interventionell radiologischen Eingriffen (z.B. Ballonangioplastie) zur Restenoseprophylaxe eingesetzt.

Die Oberfläche der bisher beschriebenen Stents ist entweder metallisch und besteht z.B. aus Edelstahl, Nitinol oder Gold, oder mit einem Polymer überzogen, wie z.B. mit Polyurethan, Polymilchsäure, Polyglycolsäure oder Mischpolymeren.

Es sind auch Stents bekannt, die mit einer Polymerschicht beschichtet sind, welche ein Therapeutikum enthält und dieses nach und nach freisetzt. Ein solcher Stent wird z.B. in der Patentanmeldung WO 91/12779 beschrieben.

In der europäischen Patentanmeldung EP 0 819 446 A2 wird ein Stent beschrieben, der mit einem Chelator beschichtet ist. Vor der Implantation wird der Stent in eine Lösung mit einem Radioisotop eingetaucht, so daß man ein radioaktives Implantat erhält. Im Gegensatz zu anderen Therapeutika soll das Radioisotop aber nicht in die Blutbahn gelangen. Bei den in dieser Anmeldung vorgeschlagenen Stents haben aber die ausgewählten Chelatoren alle schlechte komplexbildende Eigenschaften, so daß nicht gewährleistet ist, daß das Radioisotop auf dem Stent haften bleibt.

Es besteht nun das Problem, daß der Stent für den Körper einen Fremdkörper darstellt und es zu Unvertraglichkeitsreaktionen kommt. Außerdem muß bei einem radioaktiven Implantat gewährleistet sein, daß die radioaktiven Isotope fest an der Oberfläche gebunden sind und sich nicht in vivo ablösen.

Aufgabe der vorliegenden Erfindung ist es daher, Stents zur Verfügung zu stellen, die besser verträglich sind als herkömmliche Stents und bei denen Therapeutika an der Oberfläche gebunden sind. Werden als Therapeutika Radioisotope verwendet, so müssen die radioaktiven Isotope fest an der Stentoberfläche gebunden sein, damit sich die radioaktiven Ionen nicht in vivo ablösen.
Diese Aufgabe wird durch die nachfolgend beschriebenen Stents gelöst, wie sie in den Patentansprüchen gekennzeichnet sind.

### Beschreibung der Erfindung

Die oben geschilderte Aufgabe wird erfindungsgemäß dadurch gelöst, daß die Oberfläche der Stents mit einem Polymer beschichtet wird, an das hydrophile Substanzen gekoppelt sind, die zusätzlich ein Therapeutikum darstellen oder enthalten können.

Die erfindungsgemäße Vorrichtung besteht somit aus dem Grundkörper des Stents, auf den ein Polymer aufgebracht ist, welches hydrophile Substanzen trägt, die eine besondere Affinität zu Therapeutika haben.

Als Grundkörper können die handelsüblichen Implantante verwendet werden, z.B. ein Nitinol-, Edelstahl- oder Goldstent. Gebräuchlich sind der memotherm®-Stent, Wiktor-Stent, Strecker-Stent oder Palmaz-Schatz-Stent. Bevorzugt werden Nitinol-Stents verwendet.

Als Polymere kommen beispielsweise modifizierte Polyurethane in Betracht, an deren Oberfläche hydrophile Substanzen gekoppelt sind, z.B. Polyethylenglycole, Polysaccharide, Cyclodextrine oder Polyaminopolycarbonsäuren.

Die Therapeutika bilden entweder mit den hydrophilen Substanzen Komplexe (z.B. bilden radioaktive Metallionen mit DTPA sehr stabile Metallkomplexe) oder Einschlußverbindungen (z.B. bildet Cyclodextrin mit Iloprost eine sehr stabile Einschlußverbindung).

Sofern die hydrophilen Substanzen komplexbildende Eigenschaften haben, können sie Metallionen oder radioaktive Isotope fixieren. Als Beispiel für komplexbildende Chelatoren sind Polyaminopolycarbonsäuren, Kronenether, Bis-, Oligo- oder Polyphosphonate, Oligo- oder Polypeptide, Zucker wie z.B. Chitosan oder Cyclodextrinderivate zu nennen.

Polyaminopolycarbonsäuren im Sinne dieses Dokumentes sind z.B. DTPA, DOTA, DO3A, TTHA und deren Derivate. Beispielhaft genannt seien auch die Verbindungen BOPTA, Butylphenyl-DTPA, DTPA-BMEA, DTPA-BMA, Dicyclohexyl-DTPA, Dicyclohexyl-DOTA, DPDP, DTPA- oder DOTA-substituierte Polymere, GlyMeDOTA sowie GlyMeDOTA-substituierte Polymere und Porphyrinderivate.

Als radioaktive Isotope können die radioaktiven Isotope der Elemente Ag, Au, Ba, Bi, C, Co, Cr, Cu, Fe, Ga, Gd, Hg, Ho, In, Ir, Lu, Mn, P, Pb, Pd, Pm, Re, Rh, Ru, Sb, Sc, Sm, Tb, Tc oder Y verwendet werden.

Die erfindungsgemäßen Stents können beispielhaft folgendermaßen hergestellt werden:
**1. Mit Radiotherapeutika beschichtete Stents**
   1.1 Ein unbeschichteter Stent kann zunächst mit einem Polymer (z.B. ein Polyurethan, erhältlich aus der Reaktion eines amphiphilen Polyethers, Diphenylmethan-4-4'diisocyanat und Butandiol) beschichtet werden. Dieses Polymer ist derartig modifiziert, daß es an der Oberfläche Komplexbildner (z.B. DTPA-Gruppen trägt). Das Polymer wird in einem Lösemittel (z.B. Chloroform) gelöst und der Stent in die Polymerlösung eingetaucht. Nach Entnahme des Stents aus der Polymerlösung wird er in einer Trockenkammer bei Raumtemperatur getrocknet. Der hydrophile Stent ist gebrauchsfertig.
   1.2 Der nach 1.1 beschichtete Stent wird mit einer Lösung des radioaktiven Metalls (z.B. ¹¹¹InCl₃, ⁹⁰Y) versetzt. Nach Waschen des Stents ist dieser radiotherapeutisch beschichtete Stent gebrauchsfertig.
   1.3. In einer Variante dieses Verfahrens erfolgt die Beschichtung des Stents zweistufig. Dazu wird zunächst der Stent mit einem Polymer behandelt, weiches Aminogruppen tragt. Dazu liegen die Aminogruppen während der Polymerisation eventuell in geschützter Form vor. Anschließend werden die Aminogruppen mit DTPA-Monoanhydrid umgesetzt, wie es in der Literatur beschrieben ist. Der Stent weist nun eine polymere Beschichtung auf, die Komplexbildner (hier: DTPA) trägt. Der derartig beschichtete Stent wird anschließend mit einer Lösung des radioaktiven Metalls (z.B. ¹¹¹InCl₃, ⁹⁰Y) versetzt. Nach Waschen des Stents ist dieser gebrauchsfertig.
   1.3. In einer weiteren Variante des Verfahrens wird der mit dem Haftvermittler (komplexbildnerhaltiges Polymer) beschichtete Stent in ein Lebewesen implantiert. Eine Lösung eines radioaktiven Isotops wird dann intravasal appliziert. Bei diesem Verfahren findet die radioaktive Beschichtung des Stents in-vivo statt. Bei dieser Variante kann der Komplexbildneranteil des Haftvermittlers zur Steigerung der Verträglichkeit des Implantats mit physiologisch verträglichen Metallen (z.B. Natrium, Calcium, Zink, Kalium, Lithium, Magnesium) belegt sein. So können zum Beispiel Calciumionen durch die DTPA-Gruppen komplexiert sein.
**2. Mit nichtradioaktiven Therapeutika beschichtete Stents**
   2.1 Ein unbeschichteter Stent kann zunächst mit einem Polymer (z.B. ein Polyurethan, erhältlich aus der Reaktion eines amphiphilen Polyethers, Diphenylmethan-4-4'diisocyanat und Butandiol) beschichtet werden. Dieses Polymer ist derartig modifiziert, daß es an der Oberfläche Cyclodextrin trägt. Das Polymer wird in einem Lösemittel (z.B. Chloroform) gelöst und der Stent in die Polymerlösung eingetaucht. Nach Entnahme des Stents aus der Polymerlösung wird er in einer Trockenkammer bei Raumtemperatur getrocknet. Der hydrophile Stent ist gebrauchsfertig.
   2.2 Der nach 2.1 beschichtete Stent wird mit einer Lösung des Therapeutikums (z.B. Iloprost) versetzt. Das Therapeutikum bildet mit dem Cyclodextrin eine Einschlußverbindung und bleibt auf dem Stent haften. Nach Waschen des Stents ist dieser therapeutisch beschichtete Stent gebrauchsfertig.

Die oben beschriebenen Verfahren werden im allgemeinen bei Temperaturen von 0-80°C durchgeführt. Bei der Beschichtung des Stents mit dem Polymer können in Abhängigkeit von dem jeweiligen Polymer geeignete Lösemittel eingesetzt werden. Bei Einsatz eines nichtwäßrigen Lösemittels soll dieses vor der Implantation entfernt werden.

Die radioaktiven Stents können auch mit zwei oder mehr verschiedenen Isotopen beschichtet werden. Insbesondere ist es möglich, kurz- und langlebige Isotope gemeinsam auf einem Stent aufzutragen (beispielsweise ⁵⁵Co mit ⁵⁵Fe oder ⁹⁹Mo mit ⁵⁷Co).

Die nötigen Arbeitsgänge zur Durchführung der oben prinzipiell beschriebenen Verfahren sind dem Fachmann bekannt. Spezielle Ausführungsformen sind detailliert in den Beispielen beschrieben.

Ein weiteres Verfahren zur Herstellung polymerbeschichteter, radioaktiver Stents beruht auf dem in der deutschen Offenlegungsschrift DE 196 04 173 A1 offenbarten Verfahren zur Erzeugung antithrombogener Oberflächen auf medizinischen Gegenständen. Bei diesem Verfahren wird ein funktionalisiertes Polymer durch Gasphasenbeschichtung bei erhöhten Temperaturen und reduzierten Drücken auf den metallischen Stentgrundkörper aufgebracht. Wird ein aminogruppenhaltiges Polymer aufgebracht, so kann der Stent nach der Polymerbeschichtung mit einer Lösung versetzt werden, die einen Komplexbildner in reaktiver Form, z.B. DTPA-Anhydrid, enthält. Durch eine chemische Reaktion entsteht eine echte Bindung zwischen dem Polymer und dem Komplexbildner. Alternativ dazu kann der polymerbeschichtete Stent auch mit Spacermolekülen wie z.B. Diisocyanaten oder Dicarbonsäurechloriden versetzt werden, an die in einem weiteren Reaktionsschritt der Komplexbildner gebunden wird. Unter einem Spacermolekül im Sinne dieser Anmeldung ist ein Molekül zu verstehen, das zu einer chemischen Verknüpfung zwischen der Polymeroberfläche und dem Komplexbildner geeignet ist und den Effekt eines Abstandhalters bewirkt.

Die verwendeten Komplexbildner sind z.B. DTPA, DOTA, DO3A und TTHA, die alle besonders gute komplexierende Eigenschaften besitzen. Sie bilden mit Metallionen besonders stabile Komplexe, so daß nach Eintauchen eines mit Polymer und Komplexbildner beschichteten Stents in eine Lösung mit radioaktiven Metallionen diese Ionen an der Oberfläche des Stents haften bleiben. Die Stabilität der Metallkomplexe ist so gut, daß sich die Metallionen auch in vivo nicht vom Implantat lösen. Bevorzugte Isotope sind ¹⁸⁶Re, ¹⁸⁸Re, ¹¹¹In, ⁹⁰Y, ⁵⁵Co, ⁵⁷Co, ⁵⁵Fe und ⁹⁹Mo. Auch bei dieser Ausführungsform ist es möglich, mehrere Radioisotope gleichzeitig auf den Stent aufzutragen. Die Radioisotope können sowohl β- als auch γ-Strahlung emittieren.

Ferner können die erfindungsgemäßen radioaktiven Stents auch dadurch hergestellt werden, daß die Polymerschicht mit Hilfe der Plasmapolymerisation von Olefinen auf den Stentgrundkörper aufgebracht wird. Dieses Verfahren wird z.B. in der deutschen Offenlegungsschrift DE 196 47 280 A1 beschrieben. Geeignete Olefine sind z.B. Allylamin, Allylalkohol, Propargylalkohol, Butenole, Butylamine, Acrylsäure, Acrylsäurederivate, Acrylate und Hydroxymethylacrylat. An die funktionellen Gruppen der so hergestellten Polymerschicht können wiederum Komplexbildner entweder direkt oder über ein Spacermolekül gebunden werden. Auch die so hergestellten Stents werden vor Implantation bevorzugt mit Lösungen behandelt, die radioaktive Metallionen der Isotope ¹⁸⁶Re, ¹⁸⁸Re, ¹¹¹In, ⁹⁰Y, ⁵⁵Co, ⁵⁷Co, ⁵⁵Fe und/oder ⁹⁹Mo enthalten.

Es ist auch möglich, zusätzlich zu den radioaktiven Substanzen Arzneimittel wie z.B. Iloprost auf den Stent aufzubringen. Prostaglandinderivate wie Iloprost können wie oben beschrieben in Cyclodextrin-Derivate eingelagert werden, die sich auf der modifizierten Polymeroberfläche befinden.

Die erfindungsgemäßen Stents lösen die eingangs beschriebene Aufgabe. Die erfindungsgemäßen Stents sind physiologisch gut verträglich.

Komplexbildnerhaltige Stents können durch die offenbarten Verfahren problemlos und exakt dosiert radioaktiv markiert werden. Wie im Tiermodell gezeigt werden konnte, wird die Restenose nach Ballondenudation durch Implantation der erfindungsgemäßen radioaktiven Stents signifikant inhibiert.

Der besondere Vorteil der erfindungsgemäßen Stents ist, daß der Mediziner vor Ort einen Stent nach seinen Bedürfnissen auswählen und den ausgewählen Stent dann durch das beschriebene Verfahren aktivieren kann. Die Aktivierung erfolgt durch die Zugabe eines oder mehrerer radioaktiver Isotope und/oder durch das Aufbringen eines oder mehrerer Arzneimittel, die in den Träger (Chelator oder Cyclodextrin) eingelagert werden. Dadurch wird die Einstellung auf die individuellen Bedürfnisse des jeweiligen Patienten ermöglicht. Die wenigen dazu nötigen Stoffe und Lösungen können entsprechend vorbereitet angeliefert werden, so daß der entsprechende Mediziner nur noch den unbeschichteten Stent in der vorgegebenen Reihenfolge in die einzelnen Lösungen tauchen muß. Die Erfindung betrifft somit auch solche für die erfindungsgemäßen Verfahren vorbereiteten Stoffe, Lösungen und Zubereitungen (Kits).

Ein weiterer Vorteil der erfindungsgemäßen radioaktiven Stents ist, daß durch die besonders guten komplexbildenden Eigenschaften der ausgewählten Chelatoren die Radioisotope so beständig an die Polymeroberfläche gebunden werden, daß sie sich auch in vivo nicht von der Stentoberfläche ablösen und/oder durch andere Ionen ausgetauscht werden. Die Verträglichkeit der erfindungsgemäßen radioaktiven Stents ist im Vergleich zu den bekannten radioaktiven Stents wesentlich erhöht.

### Ausführungsbeispiele:

Die folgenden Beispiele sollen den Erfindungsgegenstand erläutern, ohne ihn auf diese beschränken zu wollen.

### Beispiel 1

### ¹⁸⁸Re-DTPA-beladener Stent

Als Polymer wird Polyurethan verwendet, das durch Reaktion eines amphiphilen Polyethers, Diphenylmethan-4,4'-diisocyanat und Butandiol als Kettenverlängerer erhältlich ist. Um die Ausbeute an kopplunesfähigen Gruppen zu erhöhen, können in den einzelnen Bausteinen auch zusätzliche Funktionen, wie z.B. Aminogruppen, enthalten sein, die während der Polymerisation eventuell geschützt vorliegen können. Die Stents werden dadurch beschichtet, daß sie in eine 5%ige Chloroform-Lösung des Polymers eingetaucht werden. Danach läßt man sie einer Reinraum-Trockenkammer bei Zimmertemperatur trocknen. Die durchschnittliche Schichtdicke beträgt 20 µm. Die Belegung mit dem Liganden DTPA erfolgt durch Umsetzung freier Aminogruppen mit DTPA-Monoanhydrid, wie es in der Literatur beschrieben und dem Fachmann geläufig ist. Die Komplexierung erfolgt, ebenfalls wie es dem Fachmann geläufig ist, mit einer Lösung eines Rheniumsalzes. Anschließend ist der Stent gebrauchsfertig.

### Beispiel 2

### ¹¹¹In-DTPA-Stent-Kit

Die Beschichtung des Stents mit dem Polymer und die anschließende Umsetzung mit DTPA-Monoanhydrid erfolgt wie unter Beispiel 1 beschrieben. Der Stent wird nun in dieser Form an den Radiologen ausgeliefert. Kurz vor der Applikation taucht der Radiologe den Stent in eine Lösung mit ¹¹¹In-Ionen ein, um ihn auf diese Weise zu aktivieren. Anschließend wird der Stent implantiert.

### Beispiel 3

### ¹¹¹In-DTPA-Stent-Kit

Die Beschichtung des Stents mit dem Polymer erfolgt wie unter Beispiel 1 beschrieben. Der Stent wird nun in dieser Form an den Radiologen ausgeliefert. Kurz vor der Applikation taucht der Radiologe den Stent in eine Lösung mit DTPA-Monoanhydrid ein, um den Liganden auf den Stent aufzubringen. Nach dem Herausnehmen aus der Lösung und dem Trocknen erfolgt die anschließende Umsetzung mit ¹¹¹In-Ionen. Dazu wird der Stent in eine zweite Lösung eingetaucht, die ¹¹¹In-Ionen enthält, um ihn auf diese Weise zu aktivieren. Nach dem erneuten Trocknen wird der Stent implantiert.

### Beispiel 4

### ¹¹¹In-DTPA-Stent-Kit

Die Beschichtung des Stents mit dem Polymer und die anschließende Umsetzung mit DTPA-Monoanhydrid erfolgt wie unter Beispiel 1 beschrieben. Der Stent wird nun in dieser Form an den Radiologen ausgeliefert. Nach der Applikation des Stents injiziert der Radiologe durch den Applikationskatheter eine Lösung mit radioaktiven ¹¹¹In-Ionen. Diese Lösung fließt an dem implantierten Stent vorbei und die Radioisotope werden selektiv durch die an den Stent gebundenen Liganden aus der Lösung entfernt und sind nun fest am Stent fixiert.

### Beispiel 5

Die Beschichtung eines Metallstents durch CVD-Polymerisation (CVD: Chemical Vapour Deposition) von 4-Amino-[2.2]-paracyclophan erfolgt in einer geeignet konzipierten Anlage. Die Anlage ist mit einer Argonbombe verbunden, da Argon als Trägergas fungiert. Die Argonzuleitung ist mit einem 380 mm langen Quarzglasrohr mit einem Außendurchmesser von 30 mm verbunden. Das Quarzglasrohr ist an seinem anderen Ende mit einem Edelstahlrezipienten verbunden. Das Quarzglasrohr ist frei schwebend in einem Dreizonenröhrofen gelagert, der eine beheizte Länge von 320 mm und einen Innendurchmesser von 32 mm besitzt. Alle drei Heizzonen lassen sich bis 800°C erhitzen.

Der zu beschichtende Stent wird über das abnehmbare Schauglas auf dem Probenhalter fixiert. Anschließend wird der Reaktor wieder verschlossen und die Anlage wird durch Betätigung des Hauptschalters in Betrieb genommen. Gleichzeitig werden die beiden Kühlkreisläufe aktiviert, und die Rezipientenwand wird auf 100°C geheizt. Dann wird ein Porzellanschiffchen mit einer eingewogenen Menge an Monomer in die Sublimationszone gestellt und diese wieder verschlossen. Der Reaktor wird dann auf einen Basisdruck von 0.03 mbar abgepumpt. Nun wird ein Trägergasstrom von 20 sccm eingestellt und anschließend ein Arbeitsdruck von 0.2 mbar vorgegeben. Man wartet nun so lange, bis sowohl der Trägergasfluß als auch der Arbeitsdruck konstant sind. Nun gibt man die gewünschte Pyrolysetemperatur von 680°C vor und wartet, bis diese Temperatur in der Pyrolysezone erreicht wird. Dann läßt man den Probenhalter mit einer Drehgeschwindigkeit von 20 U/min rotieren und erhitzt die Sublimationszone auf 290°C. Der Beschichtungsprozeß wird mit Hilfe des Schichtdickenmonitors verifiziert. Wenn die gewünschte Schichtdicke von 280 nm erreicht ist, kann der Beschichtungsprozeß beendet werden. Dazu werden die Ofenregler, der Drehmotor des Probenhalters und der Trägergasstrom ausgeschaltet, das Drosselventil geöffnet und noch einmal auf Basisdruck abgepumpt. Anschließend wird die Pumpe abgeschaltet, die Anlage über das Belüftungsventil belüftet und die Probe entnommen.

Zur Ankopplung von DTPA über ein Spacermolekül wird der beschichtete Stent in 500 ml einer 10 Gew.-%igen etherischen Hexamethylendiisocyanatlösung für 12 Stunden bei Raumtemperatur inkubiert. Danach wird die Probe mit Ether gewaschen und im Vakuum getrocknet. Anschließend wird der derart beschichtete Stent mit einer Lösung aus DTPA-Anhydrid in DMSO 2 Stunden bei 40°C inkubiert. Nach erneuter Reinigung wird die Oberfläche in gewohnter Weise mit ¹⁸⁸Re-Ionen beladen.

## Patentansprüche

1. Polymerbeschichteter Stent, **gekennzeichnet durch** die folgenden Merkmale: der Stent besteht aus einem Grundkörper, auf den ein Polymer aufgebracht ist, welches hydrophile Substanzen trägt, die eine besondere Affinität zu Therapeutika haben, weiterhin **dadurch gekennzeichnet, dass** der Stent zusätzlich ein oder mehrere Radioisotope ausgewählt aus den Elementen Ag, Au, Ba, Bi, Co, Cr, Cu, Fe, Ga, Gd, Hg, Ho, In, Ir, Lu, Mn, Pb, Pd, Pm, Re, Rh, Ru, Sb, Sc, Sm, Tb, Tc oder Y enthält.

2. Polymerbeschichteter Stent gemäß Anspruch 1, **dadurch gekennzeichnet, dass** das Polymer als hydrophile Substanz einen der Komplexbildner DTPA, DOTA, DO3A oder TTHA oder deren Derivate enthält.

3. Polymerbeschichteter Stent gemäß Anspruch 1, **dadurch gekennzeichnet, dass** das Polymer als hydrophile Substanz einen der Komplexbildner BOPTA, Butylphenyl-DTPA, DTPA-BMEA, DTPA-BMA, Dicyclohexyl-DTPA, Dicyclohexyl-DOTA, DPDP, ein Porhyrinderivat, ein DTPA- oder DOTAsubstituiertes Polymer, GlyMeDOTA oder ein GlyMeDOTA-substituiertes Polymer enthält.

4. Polymerbeschichteter Stent gemäß einem der Ansprüche 1 bis 3, **dadurch gekennzeichnet, dass** der Stent eines oder mehrere Radioisotope ausgewählt aus den Isotopen ¹⁸⁶Re, ¹⁸⁸Re, ¹¹¹In, ⁹⁰Y, ⁵⁵Co, ⁵⁷Co, ⁵⁵Fe und ⁹⁹Mo enthält.

5. Polymerbeschichteter Stent gemäß Anspruch 1, **dadurch gekennzeichnet, dass** der Stentgrundkörper ein Nitinol-Stent ist.

6. Polymerbeschichteter Stent gemäß Anspruch 1, **dadurch gekennzeichnet, dass** das Polymer als hydrophile Substanz Cyclodextrin enthält.

7. Polymerbeschichteter Stent nach einem der vorangehenden Ansprüche, **dadurch gekennzeichnet, dass** er ein Radioisotop und ein weiteres Therapeutikum enthält.

8. Polymerbeschichteter Stent gemäß Anspruch 7, **dadurch gekennzeichnet, dass** das Therapeutikum ein Prostaglandinderivat ist.

9. Verfahren zur Herstellung eines polymerbeschichteten Stents, **dadurch gekennzeichnet, dass** der metallische Stentgrundkörper durch Gasphasenbeschichtung bei erhöhten Temperaturen und reduzierten Drücken mit einem funktionalisierten Polymer beschichtet wird und in einem weiteren Verfahrensschritt ein Komplexbildner durch chemische Reaktion an die im Polymer vorhandenen funktionellen Gruppen gebunden wird.

10. Verfahren zur Herstellung eines polymerbeschichteten Stents, **dadurch gekennzeichnet, dass** der metallische Stentgrundkörper durch Gasphasenbeschichtung bei erhöhten Temperaturen und reduzierten Drücken mit einem funktionalisierten Polymer beschichtet wird, anschließend mit einer Lösung behandelt wird, die Spacermoleküle enthält, und schließlich mit einem weiteren Verfahrensschritt mit einer Lösung versetzt wird, die einen Komplexbildner enthält.

11. Verfahren zur Herstellung eines polymerbeschichteten Stents, **dadurch gekennzeichnet, dass** der Stentgrundkörper mit Hilfe der Plasmapolymerisation mit einem Polymer beschichtet wird und in einem weiteren Verfahrensschritt ein Komplexbildner durch chemische Reaktion an die im Polymer vorhandenen funktionellen Gruppen gebunden wird.

12. Verfahren zur Herstellung eines polymerbeschichteten Stents, **dadurch gekennzeichnet, dass** der Stentgrundkörper mit Hilfe der Plasmapolymerisation mit einem Polymer beschichtet wird, anschließend mit einer Lösung behandelt wird, die Spacermoleküle enthält, und schließlich in einem weiteren Verfahrensschritt mit einer Lösung versetzt wird, die einen Komplexbildner enthält.

13. Verfahren zur Herstellung eines polymerbeschichteten Stents, **dadurch gekennzeichnet, dass** der metallische Stentgrundkörper durch Gasphasenbeschichtung bei erhöhten Temperaturen und reduzierten Drücken mit einem funktionalisierten Polymer beschichtet wird und in einem weiteren Verfahrensschritt ein Cyclodextrinderivat durch chemische Reaktion an die im Polymer vorhandenen funktionellen Gruppen gebunden wird.

14. Verfahren zur Herstellung eines polymerbeschichteten Stents, **dadurch gekennzeichnet, dass** der metallische Stentgrundkörper durch Gasphasenbeschichtung bei erhöhten Temperaturen und reduzierten Drücken mit einem funktionalisierten Polymer beschichtet wird, anschließend mit einer Lösung behandelt wird, die Spacermoleküle enthält, und schließlich in einem weiteren Verfahrensschritt mit einer Lösung versetzt wird, die ein Cyclodextrinderivat enthält.

15. Gefäßimplantat zur Therapie und Prophylaxe von Stenosen, **dadurch gekennzeichnet, dass** das Implantat ein Stent gemäß einem der Ansprüche 1 - 8 ist.

## Claims

1. Polymer-coated stent, **characterized by** the following features: the stent consists of a main body onto which a polymer is applied which carries hydrophilic substances having a particular affinity to therapeutic agents, and further **characterized in that** the stent additionally contains one or more radioisotopes chosen from the elements Ag, Au, Ba, Bi, Co, Cr, Cu, Fe, Ga, Gd, Hg, Ho, In, Ir, Lu, Mn, Pb, Pd, Pm, Re, Rh, Ru, Sb, Sc, Sm, Tb, Tc or Y.

2. Polymer-coated stent according to Claim 1, **characterized in that** the polymer contains, as hydrophilic substance, one of the complexing agents DTPA, DOTA, DO3A or TTHA or derivatives thereof.

3. Polymer-coated stent according to Claim 1, **characterized in that** the polymer contains, as hydrophilic substance, one of the complexing agents BOPTA, butylphenyl-DTPA, DTPA-BMEA, DTPA-BMA, dicyclohexyl-DTPA, dicyclohexyl-DOTA, DPDP, a porphyrin derivative, a DTPA-substituted or DOTA-substituted polymer, GlyMeDOTA or a GlyMeDOTA-substituted polymer.

4. Polymer-coated stent according to one of Claims 1 to 3, **characterized in that** the stent contains one or more radioisotopes chosen from the isotopes ¹⁸⁶Re, ¹⁸⁸Re, ¹¹¹In, ⁹⁰Y, ⁵⁵Co, ⁵⁷Co, ⁵⁵Fe and ⁹⁹Mo.

5. Polymer-coated stent according to Claim 1, **characterized in that** the main body of the stent is a nitinol stent.

6. Polymer-coated stent according to Claim 1, **characterized in that** the polymer contains cyclodextrin as hydrophilic substance.

7. Polymer-coated stent according to one of the preceding claims, **characterized in that** it contains a radioisotope and a further therapeutic agent.

8. Polymer-coated stent according to Claim 7, **characterized in that** the therapeutic agent is a prostaglandin derivative.

9. Process for producing a polymer-coated stent, **characterized in that** the metallic main body of the stent is coated with a functionalized polymer by gas-phase coating at elevated temperatures and reduced pressures and, in a further process step, a complexing agent is bound by chemical reaction to the functional groups present in the polymer.

10. Process for producing a polymer-coated stent, **characterized in that** the metallic main body of the stent is coated with a functionalized polymer by gas-phase coating at elevated temperatures and reduced pressures, is then treated with a solution containing spacer molecules, and finally, in a further process step, has a solution containing a complexing agent added to it.

11. Process for producing a polymer-coated stent, **characterized in that** the main body of the stent is coated with a polymer with the aid of plasma polymerization and, in a further process step, a complexing agent is bound by chemcial reaction to the functional groups present in the polymer.

12. Process for producing a polymer-coated stent, **characterized in that** the main body of the stent is coated with a polymer with the aid of plasma polymerization, is then treated with a solution containing spacer molecules, and finally, in a further process step, has a solution containing a complexing agent added to it.

13. Process for producing a polymer-coated stent, **characterized in that** the metallic main body of the stent is coated with a functionalized polymer by gas-phase coating at elevated temperatures and reduced pressures, and, in a further process step, a cyclodextrin derivative is bound by chemical reaction to the functional groups present in the polymer.

14. Process for producing a polymer-coated stent, **characterized in that** the metallic main body of the stent is coated with a functionalized polymer by gas-phase coating at elevated temperatures and reduced pressures, is then treated with a solution containing spacer molecules, and finally, in a further process step, has a solution containing cyclodextrin derivative added to it.

15. Vascular implant for treatment and prevention of stenoses, **characterized in that** the implant is a stent according to one of Claims 1 - 8.

## Revendications

1. Stent revêtu de polymère, **caractérisé par** les caractéristiques suivantes : le stent est constitué d'un corps de base sur lequel est appliqué un polymère portant des substances hydrophiles qui ont une affinité particulière pour des agents thérapeutiques, de surcroît **caractérisé en ce que** le stent contient en outre un ou plusieurs radio-isotopes choisis parmi les éléments Ag, Au, Ba, Bi, Co, Cr, Cu, Fe, Ga, Gd, Hg, Ho, In, Ir, Lu, Mn, Pb, Pd, Pm, Re, Rh, Ru, Sb, Se, Sm, Tb, Tc et Y.

2. Stent revêtu de polymère selon la revendication 1, **caractérisé en ce que** le polymère contient comme substance hydrophile l'un des complexants DPTA, DOTA, DO3A et TTHA ou leurs dérivés.

3. Stent revêtu de polymère selon la revendication 1, **caractérisé en ce que** le polymère contient comme substance hydrophile l'un des complexants BOPTA, butylphényl-DTPA, DTPA-BMEA, DTPA-BMA, dicyclohexyl-DTPA, dicyclohexyl-DOTA, DPDP, un dérivé de porphyrine, un polymère substitué par DTPA ou DOTA, GlyMeDOTA ou un polymère substitué par GlyMeDOTA.

4. Stent revêtu de polymère selon l'une quelconque des revendications 1 à 3, **caractérisé en ce que** le stent contient un ou plusieurs radio-isotopes choisis parmi les isotopes ¹⁸⁶Re, ¹⁸⁸Re, ¹¹¹In, ⁹⁰Y, ⁵⁵Co, ⁵⁷Co, ⁵⁵Fe et ⁹⁹Mo.

5. Stent revêtu de polymère selon la revendication 1, **caractérisé en ce que** le corps de base du stent est un stent en nitinol.

6. Stent revêtu de polymère selon la revendication 1, **caractérisé en ce que** le polymère contient comme substance hydrophile de la cyclodextrine.

7. Stent revêtu de polymère selon l'une quelconque des revendications précédentes, **caractérisé en ce qu'**il contient un radio-isotope et un autre agent thérapeutique.

8. Stent revêtu de polymère selon la revendication 7, **caractérisé en ce que** l'agent thérapeutique est un dérivé de prostaglandine.

9. Procédé pour la fabrication d'un stent revêtu de polymère, **caractérisé en ce que** le corps de base métallique du stent est revêtu d'un polymère fonctionnalisé, par enduction en phase gazeuse à températures élevées et sous des pressions réduites et, dans une autre étape de processus, un complexant est lié par réaction chimique aux groupes fonctionnels présents dans le polymère.

10. Procédé pour la fabrication d'un stent revêtu de polymère, **caractérisé en ce que** le corps de base métallique du stent est revêtu d'un polymère fonctionnalisé, par enduction en phase gazeuse à températures élevées et sous des pressions réduites, est ensuite traité par une solution qui contient des molécules espaceurs et enfin, avec une autre étape de processus, est additionné d'une solution qui contient un complexant.

11. Procédé pour la fabrication d'un stent revêtu de polymère, **caractérisé en ce que** le corps de base du stent est revêtu d'un polymère à l'aide de la polymérisation par plasma, et, dans une autre étape de processus, un complexant est lié par réaction chimique aux groupes fonctionnels présents dans le polymère.

12. Procédé pour la fabrication d'un stent revêtu de polymère, **caractérisé en ce que** le corps de base du stent est revêtu d'un polymère à l'aide de la polymérisation par plasma, est ensuite traité par une solution qui contient des molécules espaceurs et enfin, dans une autre étape de processus, est additionné d'une solution qui contient un complexant.

13. Procédé pour la fabrication d'un stent revêtu de polymère, **caractérisé en ce que** le corps de base métallique du stent est revêtu d'un polymère fonctionnalisé, par enduction en phase gazeuse à températures élevées et sous des pressions réduites et, dans une autre étape de processus, un dérivé de cyclodextrine est lié par réaction chimique aux groupes fonctionnels présents dans le polymère.

14. Procédé pour la fabrication d'un stent revêtu de polymère, **caractérisé en ce que** le corps de base métallique du stent est revêtu d'un polymère fonctionnalisé, par enduction en phase gazeuse à températures élevées et sous des pressions réduites, est ensuite traité par une solution qui contient des molécules espaceurs et enfin, dans une autre étape de processus, est additionné d'une solution qui contient un dérivé de cyclodextrine.

15. Implant vasculaire pour la thérapie et la prophylaxie de sténoses, **caractérisé en ce que** l'implant est un stent selon l'une quelconque des revendications 1 à 8.
